(19) Europäisches Patentamt
European Patent Office
Office européen des brevets

(11) Veröffentlichungsnummer : **0 491 143 B1**

# (12) EUROPÄISCHE PATENTSCHRIFT

(45) Veröffentlichungstag der Patentschrift :
**05.05.93 Patentblatt 93/18**

(51) Int. Cl.[5] : **C07C 323/52,** C04B 41/46

(21) Anmeldenummer : **91118368.9**

(22) Anmeldetag : **29.10.91**

## (54) Gold(I)mercaptocarbonsäureester, Verfahren zu ihrer Herstellung und Verwendung.

(30) Priorität : **18.12.90 DE 4040446**

(43) Veröffentlichungstag der Anmeldung :
**24.06.92 Patentblatt 92/26**

(45) Bekanntmachung des Hinweises auf die Patenterteilung :
**05.05.93 Patentblatt 93/18**

(84) Benannte Vertragsstaaten :
**AT BE CH DE DK ES FR GB IT LI NL SE**

(56) Entgegenhaltungen :
**DE-B- 1 286 866**
**DE-B- 1 292 463**
**US-A- 4 221 826**

(73) Patentinhaber : **Degussa Aktiengesellschaft**
**Weissfrauenstrasse 9**
**W-6000 Frankfurt am Main 1 (DE)**

(72) Erfinder : **Lotze, Marion, Dr.**
**Am Galgenberg 18**
**W-6451 Hammersbach (DE)**
Erfinder : **Mehner, Hans**
**Dottenfeldstrasse 3**
**W-6230 Frankfurt 80 (DE)**

EP 0 491 143 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung richtet sich auf neue Gold(I)mercaptocarbonsäureester, welche durch eine Tricycloalkylgruppe in der Alkoholkomponente des Esters gekennzeichnet sind. Die Erfindung umfaßt ferner ein Verfahren zur Herstellung der genannten neuen Goldmercaptide und schließlich ihre Verwendung in Goldpräparaten zum Vergolden fester Unterlagen.

Zum Aufbringen eines Golddekors auf keramische Artikel und Herstellung von Leiterbahnen aus Gold in integrierten Schaltkreisen finden seit langem sogenannte Goldpräparate Anwendung. Solche Präparate enthalten im allgemeinen eine oder mehrere in einem organischen Trägermedium lösliche schwefelorganische Goldverbindungen, Flußmittel, wie beispielsweise Resinate eines oder mehrerer der Elemente B, Si, V, Cr, In, Sn, Sb, Bi und Rh, und das ein Harz und organisches Lösungsmittel enthaltende Trägermedium. Die Goldpräparate werden auf die zu beschichtende Oberfläche aufgetragen, und nach dem Abdunsten des Lösungsmittels schließt sich ein Brennvorgang bei einer auf das Substrat und das Goldpräparat abgestimmten Temperatur an, wobei der Goldfilm ausgebildet und auf der Oberfläche mit guter Haftung fixiert wird.

Bei den schwefelorganischen Goldverbindungen handelte es sich lange Zeit fast ausschließlich um sogenannte Goldsulforesinate, welche aus einem Goldsalz und geschwefelten, insbesondere natürlich vorkommenden Terpenen gewonnen wurden. Nachteilig an diesen Sulforesinaten ist, daß ihre Herstellung an natürlich vorkommende Rohstoffe, deren Vorrat begrenzt und deren Qualität Schwankungen unterworfen ist, gebunden ist. Um diesen Nachteil zu beheben und gleichzeitig die Eigenschaften der Goldpräparate gezielt zu beeinflussen, haben inzwischen rein synthetisch hergestellte Gold(I)mercaptide unterschiedlichster Struktur Eingang in die Goldpräparate gefunden.

Aus der DE-PS 12 86 866 sind definierte sekundäre Gold(I)mercaptide der Formel RR'CH-S-Au, worin R und R' Alkyl, Cycloalkyl, Aryl oder Aralkyl bedeuten, bekannt. Die Anwendung dieser Gold(I)mercaptide erforderte die Anwendung stark polarer Lösungsmittel wie Nitrobenzol. In wenig polaren Lösungsmitteln, etwa Toluol, sind teriäre Gold(I)mercaptide der Formel RR'R"C-S-Au mit R, R' und R" gleich Alkyl gemäß der DE-AS 12 98 828 löslich; diese Mercaptide weisen zudem niedrigere Zersetzungstemperaturen auf. Schließlich sind auch aromatische Gold(I)mercaptide der Formel Ar-S-Au für Vergoldungspräparate vorgeschlagen worden - DE-PS 12 84 808. Ein bicyclisches Mercaptid, nämlich Gold(I)bornylmercaptid, wurde aus der US-PS 4,221,826 bekannt; dieses Mercaptid erwies sich als vorteilhaft zur Herstellung integrierter elektronischer Schaltkreise. Nachteilig an den vorgenannten Gold(I)mercaptiden ist ihr teilweise sehr unangenehmer Geruch, der sich insbesondere dann als besonders störend bemerkbar macht, wenn die Goldpräparate in der Wärme, beispielsweise mittels Warm-Siebdruck, appliziert werden.

Aus der DE-AS 12 92 463 sind silberhaltige Vergoldungspräparate bekannt, welche eine Koordinationsverbindung aus einem Gold(I)mercaptid und einer äquimolaren Menge eines Silbercarboxylates oder Silbermercaptids enthalten. Als Gold(I)mercaptid werden primäre oder sekundäre Alkyl- oder Aralkylmercaptide, aber auch Gold(I)mercaptide des 2-Methoxyethyl-, Ethyl-, Isooctyl- und tert.-Dodecylthioglykolats genannt. Diese Koordinationsverbindungen zeichnen sich durch eine gegenüber den Einzelverbindungen höhere Löslichkeit und bessere Deckkraft aus. Nachteilig an den Koordinationsverbindungen ist, daß zwingend in äquimolarer Menge Silberverbindungen anwesend sind und damit die Dekorgestaltung hinsichtlich der Farbe eingeschränkt ist. Außerdem sind die Dekore - bedingt durch den hohen Silbergehalt - sehr anfällig gegenüber Anlauferscheinungen.

Die Verbindungsklasse der Gold(I)thioglykolsäureester weist einen wesentlich angenehmeren Geruch als die zuvor erwähnten carboxylgruppenfreien Gold(I)mercaptide auf.

Für den Einsatz in Vergoldungspräparaten, in denen eine in organischen Lösungsmitteln lösliche Goldverbindung gefordert ist, wie zum Beispiel in Glanzgoldpräparaten, sind von den in der DE-AS 12 92 463 genannten Gold(I)thioglykolsäureestern in Abwesenheit einer stöchiometrischen Menge an einer Silberverbindung nur das Gold(I)isooctylthioglykolat und das Gold(I)tert.-dodecylthioglykolat prinzipiell geeignet. Gold(I)ethylthioglykolat und Gold(I)-2-methoxyethylthioglykolat sind nahezu in organischen Lösungsmitteln unlösliche Verbindungen.

Wie die Anmelderin der vorliegenden Erfindung feststellte, sind aber die zuvor genannten Isoocyl- und tert.-Dodecyl-Gold(I)thioglykolate in Vergoldungspräparaten, insbesondere für die Dekorierung keramischer Materialien, nicht befriedigend geeignet, weil die Trocknungszeit der Präparate sehr lang ist. Damit wird die Wirtschaftlichkeit der Dekorierung aber sehr negativ beeinflußt.

Aufgabe der vorliegenden Erfindung ist, Gold(I)mercaptide aufzuzeigen, welche einen geringeren Geruch als die bisher gebräuchlichen Mercaptide ohne Esterfunktion und zusätzlich in Goldpräparaten üblicher Zusammensetzung eine geringere Lösungsmittelretention aufweisen und damit zu einer kürzeren Trocknungszeit führen als die vorbekannten Thioglykolate.

Gelöst wird die Aufgabe durch Gold(I)mercaptocarbonsäureester der allgemeinen Formel

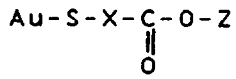 (I) ,

worin X eine Alkylengruppe und Z eine Alkylgruppe bedeuten, welche dadurch gekennzeichnet sind, daß X eine $C_1$- bis $C_3$-Alkylengruppe und Z ein Rest aus der Reihe Tricyclo[5.2.1.$0^{2,6}$]decan-8- oder -9-yl oder Tricyclo [5.2.1.$0^{2,6}$]decyl-3- oder -4-methyl ist.

Bei den erfindungsgemäßen neuen Gold(I)mercaptiden handelt es sich um Derivate der Mercaptoessigsäure, 2- und 3-Mercaptopropionsäure, 2-, 3- oder 4-Mercaptobuttersäure, 2- oder 3-Mercaptoisobuttersäure. Wegen ihrer guten Verfügbarkeit werden Produkte auf der Basis der Mercaptoessigsäure, auch Thioglykolsäure genannt, und 2- bzw. 3-Mercaptopropionsäure bevorzugt.

Die den erfindungsgemäßen Gold(I)mercaptiden zugrundeliegende Alkoholkomponente des Esters kann eine der folgenden Strukturen aufweisen:

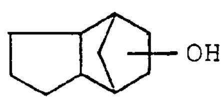 8- bzw. 9-Hydroxy-tricyclo-[5.2.1.$0^{2,6}$]decan

oder

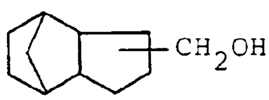 3- bzw. 4-Hydroxymethyl-tricyclo [5.2.1.$0^{2,6}$]decan.

Selbstverständlich können diese Alkohole in Form eines der möglichen Raumisomeren vorliegen. Üblicherweise werden aber Isomerengemische - Stellungs- und/oder Raumisomere - zur Herstellung der erfindungsgemäßen Gold(I)mercaptide eingesetzt, und demgemäß handelt es sich auch bei den neuen Stoffen meist um Isomerengemische.

Besonders bevorzugte erfindungsgemäße Gold(I)mercaptide sind Gold(I)mercaptoessigsäure(tricyclo [5.2.1.$0^{2,6}$]decyl-8 bzw. -9)-ester, Gold(I)mercaptoessigsäure-[(tricyclo[5.2.1.$0^{2,6}$]decyl)-3- bzw. 4-methyl]-ester, Gold(I)2- bzw. 3-mercaptopropionsäure-(tricyclo[5.2.1.$0^{2,6}$]decyl-8 bzw. -9)-ester, Gold(I)2- bzw. 3-mercaptopropionsäure-[tricyclo[5.2.1.$0^{2,6}$]decyl)-3- bzw. 4-methyl]-ester. Die neuen Stoffe lassen sich durch ihre Analyse und ihren Schmelzpunkt charakterisieren. Die Stoffe lösen sich in den üblichen Lösungsmitteln, etwa aromatischen Kohlenwasserstoffen, welche in Trägermedien für Vergoldungspräparate zur Anwendung gelangen.

Durch die neuen Gold(I)mercaptide wurde die Palette an Goldverbindungen, die sich hervorragend zum Einsatz in Goldpräparaten eignen, erweitert. Der besondere Vorteil der neuen Gold(I)mercaptide besteht darin, daß die Trocknungszeit bei ansonsten vergleichtbarer Zusammensetzung des Präparates auf einen Bruchteil der für die vorbekannten Gold(I)mercaptoessigsäureester erforderlichen Trocknungszeit verkürzt werden konnte. Diese Trocknungszeitverkürzung, welche in einzelnen Fällen um/über 90 % betragen kann, war nicht vorhersehbar. Die neuen Gold(I)mercaptide weisen nur einen schwachen Geruch auf und bereiten damit in arbeitshygienischer Hinsicht bei ihrer Anwendung keine Probleme. Durch die Auswahl und Kombinationsmöglichkeit der Gruppen X und Z ist es möglich, die Eigenschaften der Gold(I)mercaptide zu modifizieren und näher der gewünschten Anwendung anzupassen: eine solche Anpassung war bei den Gold(I)bornylmercaptiden der US-PS 4,221,826 nicht möglich.

Die neuen Gold(I)mercaptide der Formel (I) lassen sich in an sich bekannter Weise, wie sie aus der Herstellung vorbekannter Gold(I)mercaptide bekannt ist, herstellen - vgl. US-PS 4,221 ,826, DE-AS 12 86 866, DE-AS 12 98 828:

Ein Gold(I)chlorid-Dialkylsulfidkomplex, zugänglich in bekannter Weise durch Umsetzung einer wäßrigen Lösung von Tetrachlorogoldsäure mit der zweifach stöchiometrischen Menge eines Dialkylsulfids in Gegenwart von Wasser, wird mit einem Mercaptocarbonsäureester der allgemeinen Formel

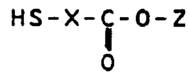 (II) ,

worin X und Z die gleiche Bedeutung haben wie in Formel (I), im Molverhältnis von im wesentlichen 1 zu 1 in Gegenwart eines organischen Lösungsmittels bei 0 bis 40 °C umgesetzt. Das organische Lösungsmittel soll den Ester der Formel (II) lösen, praktisch aber nicht das gewünschte Gold(I)mercaptid der Formel (I). Unter einem Molverhältnis von im wesentlichen 1 zu 1 wird verstanden, daß ein geringer Überschuß - bis zu 10 Mol-% - des Esters der Formel (II) anwesend sein kann, ein Molverhältnis von 1 zu 1 wird aber bevorzugt. Als Gold(I)chlorid-Dialkylsulfidkomplex, der als solcher oder in Form des Reaktionsgemisches aus seiner Herstellung eingesetzt werden kann, kommt einer der vorbekannten Dialkylsulfidkomplexe, etwa der Dimethyl- oder Diethylsulfidkomplex infrage, vorzugsweise wird aber der Gold(I)-Methioninkomplex verwendet. Bei der Umsetzung des genannten Goldkomplexes mit dem Ester der Formel (II) fällt das Gold(I)mercaptid der Formel (I) aus. Unter Verwendung von Methylenchlorid als organisches Lösungsmittel gelingt es besonders gut, ein pulverförmiges Produkt zu erhalten, das sich in einfacher Weise säurefrei waschen läßt und vor allem keine Einschlüsse des eingesetzten Gold(I)-Dialkylsulfidkomplexes und Mercaptocarbonsäureesters enthält. An die eigentliche Umsetzung schließen sich die Fest-Flüssig-Phasentrennung, danach eine Wäsche des Feststoffs mit Wasser oder wäßrigen Lösungen zur Entfernung des gebildeten Chlorwasserstoffs und schließlich die Trocknung an. Zweckmäßigerweise wird vor der Trocknung anhaftendes Wasser durch Waschen mit einem Gemisch aus Methanol oder Ethanol und Methylenchlorid oder einem anderen niedrigsiedenden aliphatischen Chlorkohlenwasserstoff entfernt. Die Trocknung erfolgt vorzugsweise bei Temperaturen unter 50 °C.

Die Ester der Formel (II) lassen sich mittels üblicher säurekatalysierter Veresterungsmethoden aus den Mercaptocarbonsäuren und den tricyclischen Alkoholen der vorgenannten Struktur in Gegenwart eines Azeotropschleppmittels gewinnen. Ein besonders geeignetes Schleppmittel ist Toluol; eine Reinigung des Esters der Formel (II) zur Herstellung der Gold(I)mercaptide der Formel (I) ist im allgemeinen nicht erforderlich.

Wie bereits hervorgehoben, lassen sich die erfindungsgemäßen Gold(I)mercaptocarbonsäureester der Formel (I) in Goldpräparaten zum Vergolden fester Unterlagen, insbesondere aus keramischen Materialien, verwenden. Unter Goldpräparaten werden flüssige bis pastenförmige Präparate verstanden, welche nach verschiedenen Auftragemethoden, wie beispielsweise Pinselauftrag, Spritzen, Direkt- und Indirektdruck, Warm- oder Kaltsiebdruck appliziert werden. Die zu vergoldenden Unterlagen müssen eine ausreichende thermische Beständigkeit aufweisen, um dem Brennvorgang, bei welchem sich der Goldfilm ausbildet, zu widerstehen. Die Brenntemperaturen können im Bereich von etwa 200 bis über 1000 °C liegen, jedoch werden im Falle von keramischen Oberflächen Temperaturen im Bereich von 400 bis 900 °C bevorzugt.

Goldpräparate zum Vergolden keramischer Materialien, wie Glas, Glaskeramik, Porzellan und andere silikatische oder nichtsilikatische keramische Werkstoffe, enthalten außer der löslichen Goldverbindung noch Flußmittel und das organische Trägermedium aus Harzen und Lösungsmitteln. Zusätzlich können weitere Verarbeitungshilfsstoffe sowie das Aussehen und die Eigenschaften des Goldfilms beeinflussende Stoffe, wie z. B. andere lösliche Edelmetallverbindungen, Goldpulver und Glasfritten, enthalten sein. Der Gehalt an Gold in den zu Dekorationszwecken sowie zu technischen Zwecken, wie gedruckte Schaltungen und Lampenreflektoren, geeigneten Goldpräparaten kann in weiten Grenzen liegen. Sogenannte Glanzgoldpräparate enthalten meist 8 bis 12 Gew.-% Gold in Form einer oder mehrerer Gold(I)mercaptide; Poliergoldpräparate enthalten außer den Goldverbindungen elementares Gold - Gesamt-Goldgehalt meist 15 bis 40 Gew.-%. Wiederum eine andere Klasse von Goldpräparaten, wofür die erfindungsgemäßen Gold(I)mercaptide der Formel (I) Einsatz finden können, sind die sogenannten Goldlüsterpräparate, welche nur einen niedrigen Goldgehalt von wenigen Gew.-%, daneben aber mehr Goldflußmittel enthalten und keinen zusammenhängenden Goldfilm, sondern ein Dekor mit einem charakteristischen goldenen Lüsterglanz ergeben.

Bei den Flußmitteln handelt es sich um solche, wie sie einleitend genannt und in den dort zitierten Dokumenten näher beschrieben worden sind, also insbesondere um Sulforesinate, Resinate, Naphthenate, Carboxylate und Dithiocarbamate der Elemente B, Si, V, Cr, In, Sn, Pb, Sb, Bi sowie Rh. Typische Trägermedien enthalten ein oder mehrere organische Lösungsmittel aus der Reihe von beispielsweise Ketonen, aromatischen und aliphatischen Kohlenwasserstoffen, aliphatischen Chlorkohlenwasserstoffen, Alkylacetaten, Glykolethern, Terpenkohlenwasserstoffen und ätherischen Ölen sowie Wachsen im Falle von Medien für den Warm-Siebdruck und ein oder mehrere Harze aus der Reihe von beispielsweise Holzharzen, wie z. B. Kolophonium- und Dammarharz, und synthetischen Harzen, wie z. B. Kohlenwasserstoffharze und Polyacrylate bzw. Polymethacrylate. Typische Glanzgoldpräparate für den Siebdruck enthalten 20 bis 30 Gew.-% der erfindungsgemäßen Gold(I)mercaptide - als reine Verbindung oder Isomerengemisch -, Flußmittel, insbesondere Gemische aus Cr-, Bi- und Rh-Resinaten, in einer Menge von 0, 1 bis 0,5 Gew.-%, berechnet als Chromoxid, Wismutoxid und Rhodiummetall, und 65 bis 80 Gew.-% Trägermedium, wovon etwa ein Viertel auf Harze entfällt.

Die Trocknungsgeschwindigkeit des Goldpräparates hängt bei gleichen externen Bedingungen - Temperatur, Druck, relative Luftfeuchte - von der absoluten Verdunstungsgeschwindigkeit der anwesenden Lösungsmittel und vom Ausmaß der Lösungsmittelretention durch die organischen Bestandteile des Präparates, also

auch des eingesetzten Gold(I)mercaptids, ab. Es hat sich gezeigt, daß, ohne die gewünschten Verarbeitungseigenschaften eines Präparates, die im wesentlichen durch die Art des Trägermediums bestimmt werden, zu beeinträchtigen, die Trocknungseigenschaften in unerwartet starkem Ausmaß von der Struktur der organischen Goldverbindung beeinflußt werden. Die Struktur der erfindungsgemäßen Stoffe führt zu außergewöhnlich kurzen Trocknungzeiten im Vergleich zu strukturähnlichen vorbekannten Stoffen - siehe Beispiele 3 und 4 und Vergleichsbeispiele 1 und 2. Innerhalb der Gruppe der erfindungsgemäßen Stoffe der Formel (I) sind die Trocknungszeiten etwa gleich.

Ermittlung der Trocknungszeit: Es werden Flächen von 5 x 5 cm mit dem Goldpräparat bedruckt (Siebdruck); in Abständen von einer halben bis einer Stunde wird die Fläche mit Seesand überschichtet; der Trocknungsvorgang ist beendet, wenn beim Senkrechtstellen der übersandeten Fläche der Sand aufgrund mangelnder Haftung abfällt.

**Beispiel 1**

Herstellung der Mercaptocarbonsäureester der Formel (II)

Umsetzung Mercaptoessigsäure mit 3(4)-Hydroxymethyl-tricyclo[5.2.1.$0^{2,6}$]decan:

1,2 mol 98 %ige Mercaptoessigsäure (112,7 g) und 1 mol 3(4)-Hydroxymethyl-tricyclo[5.2.1.$0^{2,6}$]decan (166.0 g) (Isomerengemisch "TCD-Alkohol M" / Fa. Hoechst), gelöst in 250 ml Toluol, werden in Gegenwart von 2 g p-Toluolsulfonsäure am Wasserabscheider unter Rückfluß (ca. 120 °C) erhitzt. Nach ca. 2,5 h ist die theoretische Menge an Wasser (18 ml) abgeschieden. Nach Abkühlen wird das Reaktionsgemisch zur Abtrennung der überschüssigen Mercaptoessigsäure mit 10 gew.-/%iger Natronlauge (ca. 80 ml) unter Rühren (ca. 30 min) neutralisiert. Die wäßrige Phase wird abgetrennt und das Toluol unter vermindertem Druck (15 mbar, 40 bis 120 °C) abdestilliert. Zurück bleiben 247,5 g einer farblosen Flüssigkeit mit einem Restgehalt an Toluol von 3 Gew.-%. Die Umsetzung verläuft quantitativ. Der rohe Mercaptoessigsäure-[(tricyclo[5.2.1.$0^{2,6}$]decyl)-3(4)methyl]ester wird ohne weitere Reinigung weiter umgesetzt. Identifizierung: $^1$H-NMR-Spektren.

In prinzipiell gleicher Weise wurden weitere Produkte der Formel (II) hergestellt, wobei die jeweilige Mercaptocarbonsäure und der oben genannte "TCD-Alkohol M" bzw. 8(9)-Hydroxytricyclo[5.2.1.$0^{2,6}$]decan (Isomerengemisch "TCD-Alkohol A" der Fa. Hoechst) eingesetzt wurde.

**Beispiel 2**

a) Herstellung von Goldomercaptoessigsäure-[(tricyclo[5.2.1.$0^{2,6}$]decyl)-3(4)methyl]-ester

1 mol Gold in Form einer wäßrigen Lösung von Tetrachlorogoldsäure (38 Gew.-% Au) (518.5 g) werden innerhalb einer Stunde in eine Suspension von 2 mol D, L-Methionin (298,4 g) und 2 l Wasser getropft. Die Temperatur wird durch externe Kühlung auf 0 bis 5 °C gehalten. Nach Beendigung der Reduktion liegt der Gold(I)chlorid-D,L-Methioninhydrochlorid-Komplex - teils gelöst, teils als Niederschlag - vor. Bei einer Temperatur von 5 bis 15 °C werden innerhalb einer Stunde zu dieser Suspension 1 mol des Mercaptoessigsäure-[(tricyclo[5.2.1.$0^{2,6}$]decyl)-3(4)-methyl]-esters (240,1 g), vermischt mit 250 ml Dichlormethan und 250 ml Ethanol, zugetropft. Der Goldmercaptoessigsäure-[(tricylco[5.2.1.$0^{2,6}$]decyl)-3(4)-methyl]-ester fällt als weißer Niederschlag an, der sofort nach dem Zutropfen abfiltriert wird. Der Niederschlag wird zur Reinigung 30 min lang in 0,8 l Dichlormethan gerührt, mit 1 ,5 l Ethanol versetzt, 15 min lang gerührt und abgesaugt. Anschließend wird das Rohprodukt 30 min lang mit 450 g 3 %igem Natriumhydrogencarbonat gerührt, abgesaugt und auf dem Filter mit 500 ml Wasser gewaschen. Das Rohprodukt wird erneut 30 min lang in 0,8 l Dichlormethan gerührt, mit 1,5 l Ethanol versetzt und 15 min lang gerührt und abgesaugt. Das Rohprodukt wird in dünnen Lagen bei 50 °C 24 Stdn. getrocknet. Die Ausbeute liegt bei 98 %, bezogen auf das eingesetzte Gold.

| | | | | |
|---|---|---|---|---|
| Schmelzpunkt | 172 °C | | | |
| Analyse: | Au | C | H | S |
| ber: | 45,14 | 35,79 | 4,39 | 7,35 |
| gef: | 44,92 | 35,68 | 4,32 | 7,30 |

In analoger Weise, wie in Beispiel 2 beschrieben, wurden die nachfolgenden Goldmercaptide aus den entsprechenden Mercaptocarbonsäureestern hergestellt

b)

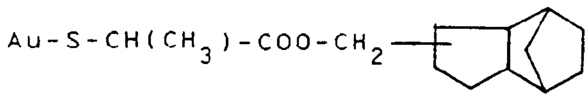

| | | | | |
|---|---|---|---|---|
| Schmelzpunkt | 108 °C | | | |
| Analyse: | Au | C | H | S |
| ber: | 43,74 | 37,34 | 4,70 | 7,12 |
| gef: | 43,68 | 37,40 | 4,68 | 7,11 |

c)

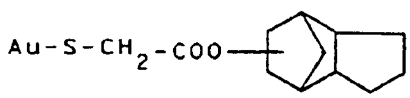

Au (theoretisch): 46,64 %
Au (experimentell): 46,48 %
Schmelzpunkt: 138 °C

d)

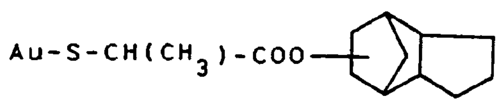

Au (theoretisch): 45,14 %
Au (experimentell): 44,91 %
Schmelzpunkt: 97 °C

**Beispiel 3 und Vergleichsbeispiel 1**

Glanzgoldpräparate (erfindungsgemäß und nach dem Stand der Technik) - Zusammensetzung (Angaben in Gew.-%) und Trocknungszeiten (Stdn.):

| | | | |
|---|---|---|---|
| A | 54,13 | 24,39<br>29,74 | Goldmercaptoessigsäure-(2-ethylhexyl)-ester in<br>Mesitylen |
| B | 54,13 | 26,55<br>27,58 | Goldmercaptoessigsäure-[(tricyclo[5.2.1.$0^{2,6}$]decyl)-3(4)-methyl]-ester in<br>Mesitylen |
| C | 1,00<br>1,00<br>1,50<br>20,00<br>22,37 | | Rhodiumsulforesinat in Terpeniol, 5 % Rh<br>Wismutresinat in Terpeniol, 7 % $Bi_2O_3$<br>Chromresinat in Terpeniol, 3 % $Cr_2O_3$<br>geschwefeltes Kolophoniumharz<br>Mesitylen |

Die Mischungen aus A plus C (Vergleichsbeispiel 1) und B plus C (Beispiel 3) wurden auf Glasplatten im Siebdruck aufgetragen (Polyester-Siebgewebe "110 T" = 110 Fäden pro cm, mittelschwer).

Die Trocknung erfolgte bei 20 °C und einer rel. Luftfeuchtigkeit von 60 %.

Die Glanzgoldpaste, bestehend aus den Komponenten A und C erforderte eine Trocknungszeit von 26 Stunden.

Die Glanzgoldpaste, bestehend aus den Komponenten B und C (erfindungsgemäß) benötigte nur eine Trocknungszeit von 2 Stunden.

**Beispiel 4 und Vergleichbeispiel 2**

Glanzpalladiumpräparate (erfindungsgemäß und nach dem Stand der Technik ) - Zusammensetzung (Gew.-%) und Trocknungszeiten (Stdn.)

| | | | |
|---|---|---|---|
| A | 50,32 | 20,32 | Goldmercaptoessigsäure-(2-ethylhexyl)-ester in |
| | | 30,00 | Mesitylen |
| B | 50,32 | 21,44 | 2-Goldmercaptopropionsäure-tricyclo[5.2.1.0$^{2,6}$]decyl-8(9))-ester in |
| | | 28,88 | Mesitylen |
| C | 1,00 | | Rhodiumsulforesinat in Terpeniol, 5 % Rh |
| | 1,00 | | Wismutresinat in Terpeniol, 7 % $Bi_2O_3$ |
| | 1,50 | | Chromresinat in Terpeniol, 3 % $Cr_2O_3$ |
| | 6,30 | | Palladiumsulforesinat in Terpeniol, 8 % Pd |
| | 5,00 | | Silbersulforesinat, 20 % Ag |
| | 17,00 | | geschwefeltes Kolophoniumharz |
| | 17,88 | | Mesitylen |

Gemäß Beispiel 3 / Vergleichsbeispiel 1 wurden die Mischungen aus den Komponenten A plus C (Vergleichsbeispiel 2) und B plus C (Beispiel 4) per Siebdruck aufgetragen und die Trocknungszeit bestimmt.

**Vergleichsbeispiel 2:** Trocknungszeit 18 h
**Beispiel 4:** Trocknungszeit 1 h

## Patentansprüche

1. Gold(I)mercaptocarbonsäureester der allgemeinen Formel

$$\mathrm{Au-S-X-\underset{\underset{O}{\|}}{C}-O-Z} \qquad (I),$$

worin X eine Alkylengruppe und Z eine Alkylgruppe bedeuten,
dadurch gekennzeichnet,
daß X eine $C_1$ - bis $C_3$-Alkylengruppe, und Z ein Rest aus der Reihe Tricyclo[5.2.1.0$^{2,6}$]decan-8- oder -9-yl oder Tricyclo [5.2.1.0$^{2,6}$]decyl-3- oder -4-methyl ist.

2. Gold(I)mercaptocarbonsäureester nach Anspruch 1,
dadurch gekennzeichnet,
daß X die Gruppe $CH_2$ oder $CH(CH_3)$ ist.

3. Verfahren zur Herstellung von Gold(I)mercaptocarbonsäureestern des Anspruch 1 durch Umsetzen eines Gold(I)chlorid-Dialkylsulfidkomplexes mit einem Mercaptocarbonsäureester der allgemeinen Formel

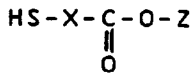

(II) ,

im Molverhältnis von im wesentlichen 1 zu 1 in Gegenwart eines organischen Lösungsmittels, das den Mercaptocarbonsäureester löst, den Gold(I)mercaptocarbonsäureester der Formel (I) aber praktisch nicht löst, bei 0 bis 40 °C, Abtrennen des ausgefallenen Gold(I)mercaptocarbonsäureesters, Nachwaschen desselben mit Wasser und Trocknen,
dadurch gekennzeichnet,
daß man einen Mercaptocarbonsäureester der Formel (II) verwendet, in welchem X eine $C_1$- bis $C_3$-Alkylengruppe und Z ein Rest aus der Reihe Tricyclo[5.2.1.$0^{2,6}$)decan-8- oder -9-yl oder Tricyclo[5.2.1.$0^{2,6}$]decyl-3- oder -4-methyl ist.

4. Verfahren nach Anspruch 3,
dadurch gekennzeichnet,
daß man als organisches Lösungsmittel Methylenchlorid verwendet und X die Gruppe $CH_2$, $CH_2$-$CH_2$ oder $CH(CH_3)$ bedeutet.

5. Verwendung der Gold(I)mercaptocarbonsäureester der Formel (I) in Goldpräparaten zum Vergolden fester Unterlagen, insbesondere aus keramischen Materialien.

## Claims

1. An ester of a gold(I) mercaptocarboxylic acid of the general formula

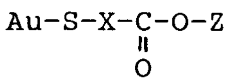

(I)

in which X represents an alkylene group and Z represents an alkyl group,
characterised in that
X is a $C_1$- to $C_3$-alkylene group and Z is a radical from the tricyclo[5.2.1.$0^{2,6}$]decane-8 or -9-yl or tricyclo[5.2.1.$0^{2,6}$]decyl-3- or -4-methyl series.

2. An ester of a gold(I) mercaptocarboxylic acid according to Claim 1,
characterised in that
X is the $CH_2$ or $CH(CH_3)$ group.

3. A process for the preparation of esters of gold(I) mercaptocarboxylic acids as in Claim 1 by reacting a gold(I) chloride/dialkyl sulphide complex with an ester of a mercaptocarboxylic acid of the general formula

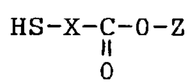

(II)

in the molar ratio of essentially 1 to 1 in the presence of an organic solvent which dissolves the ester of a mercaptocarboxylic acid, but which virtually does not dissolve the ester of a gold(I) mercaptocarboxylic acid of the formula (I), at 0 to 40°C, separating out the precipitated ester of the gold(I) mercaptocarboxylic acid, washing this with water and drying,
characterised in that
an ester of a mercaptocarboxylic acid of the formula (II) is used in which X is a $C_1$- to $C_3$-alkylene group and Z is a radical from the tricyclo[5.2.1.$0^{2,6}$]decane-8- or -9-yl or tricyclo[5.2.1.$0^{2,6}$]decyl-3- or -4-methyl series.

4. A process according to Claim 3,
characterised in that
methylene chloride is used as the organic solvent and X represents the $CH_2$, $CH_2$-$CH_2$ or $CH(CH_3)$ group.

5. Use of the ester of a gold(I) mercaptocarboxylic acid of the formula (I) in gold preparations for gilding solid substrates, especially made from ceramic materials.

## Revendications

1. Esters d'acides auro(I)mercaptocarboxyliques de formule générale :

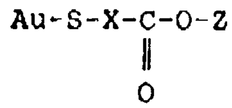

(I)

dans laquelle X signifie un groupe alcoylène et Z signifie un radical alcoyle, caractérisé en ce que X est un groupe alcoylène en $C_1$ à $C_3$, et Z est un reste choisi dans la série formée du tricyclo [5.2.1.$0^{2,6}$]decane-8 ou -9- yl ou du tricyclo-[5.2.1.$0^{2,6}$]decyl-3 ou-4-méthyle.

2. Ester d'acide auro(I)mercaptocarboxylique selon la revendication 1, caractérisé en ce que X est un groupe $CH_2$ ou $CH(CH_3)$.

3. Procédé d'obtention des esters d'acide auro(I)mercaptocarboxylique selon la revendication 1 par réaction d'un complexe sulfure de dialcoyle/chlorure d'or (I) avec un ester d'acide mercaptocarboxylique de formule générale :

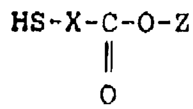

(II)

dans un rapport molaire essentiellement de 1 à 1 en présence d'un solvant organique, qui dissout l'ester d'acide mercaptocarboxylique, mais qui ne dissout pratiquement pas l'ester d'acide auro(I) mercaptocarboxylique de formule (I), de 0 à 40° C, séparation de l'ester d'acide auro(I)mercaptocarboxylique qui a précipité, post-lavage de celui-ci avec de l'eau et séchage, caractérisé en ce que l'on utilise un ester d'acide mercaptocarboxylique de formule (II), dans laquelle X est un groupe alcoylène en $C_1$ à $C_3$, et Z est un reste choisi dans la série formée du tricyclo [5.2.1.$0^{2,6}$] décane-8- ou 9-yl ou du Tricyclo [5.2.1.$0^{26}$] décyl-3 ou -4 méthyle.

4. Procédé selon la revendication 3, caractérisé en ce que l'on utilise comme solvant organique du chlorure de méthylène et que X signifie le groupement $CH_2$, $CH_2$-$CH_2$ ou $CH(CH_3)$.

5. Utilisation des esters d'acide auro(I)-mercaptocarboxylique de formule (I) dans des préparations d'or pour la dorure de supports solides, en particulier en matériaux céramiques.